# EUROPEAN PATENT SPECIFICATION

(11) **EP 2 763 663 B1**
(45) Date of publication and mention of the grant of the patent: **27.12.2017**
(21) Application number: 12766990.1
(22) Date of filing: 03.10.2012
(51) Int. Cl.: A61K 9/20, A61K 9/50, A61K 31/436

(54) **PHARMACEUTICAL COMPOSITIONS COMPRISING 40-O-(2-HYDROXY)ETHYL-RAPAMYCIN**
PHARMAZEUTISCHE ZUSAMMENSETZUNGEN MIT 40-O-(2-HYDROXY)ETHYL-RAPAMYCIN
COMPOSITIONS PHARMACEUTIQUES COMPRENANT 40-O-(2-HYDROXY)ÉTHYL-RAPAMYCINE

(30) Priority: 06.10.2011 US 201161544026 P
(43) Date of publication of application: 13.08.2014
(73) Proprietor: Novartis AG, 4056 Basel (CH)
(72) Inventor: DIEDERICH, Anke, 4002 Basel (CH); LIECHTI, Kurt, 4002 Basel (CH); KUEHL, Peter, 4002 Basel (CH); CHEUNG, Wing, East Hanover New Jersey 07936-1080 (US)
(74) Representative: Tostmann, Holger Carl
(86) International application number: PCT/EP2012/069541
(87) International publication number: WO 2013/050419

(56) References cited:
- WO-A1-2005/034916
- WO-A2-2010/056754

## Description

The present invention relates to solid pharmaceutical formulations comprising 40-O-(2-hydroxy)ethyl-rapamycin, to solid dosage forms which comprise said solid pharmaceutical formulations, to the formulation for use in a method involving a patient and to use of the formulation and solid dosage forms as a medicine. Methods of preparing said solid pharmaceutical formulations and said solid dosage forms, uses of said solid pharmaceutical formulations and said solid dosage for the manufacture of a medicament for the treatment or prevention of diseases or conditions responsive to inhibition of mTOR signalling pathway, such as for instance proliferative diseases or immunosuppression are also disclosed herein.

Rapamycin is a lactam macrolide antibiotic produced by Streptomyces hygroscopicus. Rapamycins are potent immunosuppressants and have antitumor and antifungal activity. However, its utility as pharmaceutical is restricted by its very low and variable bioavailability. Moreover, rapamycin is poorly soluble in aqueous media, e.g. water, making it difficult to formulate galenic compositions.

40-O-(2-hydroxy)ethyl-rapamycin (everolimus, RAD001) is an orally active rapamycin derivative which is described for instance in Example 8 of WO94/09010. 40-O-(2-hydroxy)ethyl-rapamycin has been first approved as immunosuppressant in 2003 and is available to patients now in > 80 countries under the name of Certican© / Zortress©, e.g. for the prevention of organ rejection, or under the name Afinitor© / Votubia© for the treatment of tumor diseases.

In a first aspect the invention provides an extended release pharmaceutical formulation for oral administration in form of a multiparticulate comprising a) 40-O-(2-hydroxy)ethyl-rapamycin, b) at least one extended release coating which comprises i) a water insoluble coating forming polymer and ii) optionally a pore former, and c) a protection layer, wherein the protection layer separates the layer comprising the 40-O-(2-hydroxy)ethyl-rapamycin from the extended release coating.

In a second aspect the invention provides a solid dosage form comprising an extended release pharmaceutical formulation according to the first aspect above in the form a minitablet, pellets, microparticles, granules or beads.

In another aspect the invention provides the extended release pharmaceutical formulation or the solid dosage form according to the aforementioned aspects for use as a medicine.

In yet another aspect the invention provides extended release pharmaceutical formulation for oral administration in a form of multiparticulates for use in a method of reducing everolimus Cmax to Cmin ratio in a patient according to the first aspect.

Further embodiments are set forth in the dependent claims.

40-O-(2-hydroxy)ethyl-rapamycin is a macrolide of low solubility in water and low chemical stability. On oral administration to humans, solid O-(2-hydroxy)ethyl-rapamycin may not be absorbed in sufficient amount into the blood stream. Mixtures of O-(2-hydroxy)ethyl-rapamycin with conventional pharmaceutical excipients can lead to instability; disadvantages with such compositions include unpredictable dissolution rates or irregular bioavailability. 40-O-(2-hydroxy)ethyl-rapamycin formulations and methods for the preparation of such formulations are e.g. disclosed in WO97/03654 relating to oral pharmaceutical compositions for rapamycins, such as for instance 40-O-(2-hydroxy)ethyl-rapamycin, which are in the form of a solid dispersion. WO03/028705 discloses oral pharmaceutical compositions for rapamycins, such as for instance 40-O-(2-hydroxy)ethyl-rapamycin, comprising colloidal silicon dioxide to promote disintegration. WO05/034916 describes inter alia pharmaceutical compositions for fixed-dose combinations comprising MMF/MMA and RAD001 which are in multiparticulate form and wherein the MMF/MMA particles are preferably enterically coated. The disclosed delayed release enteric coating comprises pH dependent polymers comprising carboxy groups such as cellulose acetate phthalate; cellulose acetate trimellitate; methacrylic acid copolymers, e.g. copolymers derived from methylacrylic acid and esters thereof, containing at least 40% methylacrylic acid; hydroxypropyl methylcellulose phthalate; and hydroxypropylmethylcellulose acetate succinate. Such pH dependent polymers typically are not compatible with stable RAD001 if formulated together in one multiparticulate subunit or monolithic dose unit.

40-O-(2-hydroxy)ethyl-rapamycin is available in solid dosage forms for oral administration as 0.1 to 10 mg immediate release tablets. However, still today it is difficult to formulate 40-O-(2-hydroxy)ethyl-rapamycin as oral solid dosage forms which meet both the requirements of satisfying drug product stability and sufficient oral bioavailability at the same time. 40-O-(2-hydroxy)ethyl-rapamycin is moisture labile, incompatible to many commonly used excipients as well as sensitive to light and oxidative stress. Thus, specific measurements are required to stabilize the drug substance during processing and throughout the shelf life time of the drug product. Furthermore, solubility enhancing principles have to be applied for ensuring consistent, reliable drug absorption with low variability and degradation of O-(2-hydroxy)ethyl-rapamycin in the gastro-intestinal tract needs to be minimized for optimizing the drug efficacy and for reducing variability of absorption in and/or among patients.

The present invention now provides improved pharmaceutical formulations in form of oral solid dosage forms comprising 40-O-(2-hydroxy)ethyl-rapamycin which satisfy product stability requirements and have favorable pharmacokinetic properties over the current IR tablets, such as reduced average plasma peak concentrations, reduced inter- and intra-patient variability in the extent of drug absorption and in the plasma peak concentration, reduced Cₘₐₓ / Cₘᵢₙ ratio and reduced food effects. The improved solid formulation of the present invention allow for more precise dose adjustment and reduce frequency of adverse events thus providing safer treatments for 40-O-(2-hydroxy)ethyl-rapamycin to the patients.

The present invention relates to stable extended release formulations of 40-O-(2-hydroxy)ethyl-rapamycin which are multiparticulate systems and may have functional layers and coatings.

In one aspect, the present invention provides stable extended release formulations comprising 40-O-(2-hydroxy)ethyl-rapamycin, as active ingredient formulated as extended release, multiparticulate formulation. The term "extended release, multiparticulate formulation as used herein refers to a formulation which enables release of 40-O-(2-hydroxy)ethyl-rapamycin over an extended period of time e.g. over at least 1, 2, 3, 4, 5 or 6 hours. The extended release formulation contains matrices and coatings made of special excipients, e.g. as described hereinbelow, which are formulated in a manner as to make the active ingredient available over an extended period of time following ingestion.

For the purpose of the present invention, the term "extended release" can be interchangeably used with the terms "sustained release" or "prolonged release". The term "extended release" relates to a pharmaceutical formulation that does not release active drug substance immediately after oral dosing but over an extended in accordance with the definition in the pharmacopoeias Ph. Eur. (7th edition) mongraph for tablets and capsules and USP general chapter <1151> for pharmaceutical dosage forms. The term "Immediate Release" as used herein refers to a pharmaceutical formulation which releases 85% of the active drug substance within less than 60 minutes in accordance with the definition of "Guidance for Industry: "Dissolution Testing of Immediate Release Solid Oral Dosage Forms" (FDA CDER, 1997). Specifically the term "immediate release" means release of everolismus from tablets within the time of 30 minutes, e.g. as measured in the dissolution assay described hereinbelow.

The pharmaceutical formulations according to the present inventions can be characterized by an in-vitro release profile using a dissolution assay as described hereinbelow: a dissolution vessel filled with 900 mL phosphate buffer pH 6.8 containing sodium dodecyl sulfate 0.2% at 37°C and the dissolution is performed using a paddle method at 75 rpm according to USP by according to USP testing monograph 711, and Ph.Eur. testing monograph 2.9.3. respectively.

The extended release formulations according to the present invention typically release 40-O-(2-hydroxy)ethyl-rapamycin in the in-vitro release assay according to following release specifications:
0.5h: <45%, or <40, preferably: <30%
1h: 20-80%, preferably: 30-60%
2h: >50%, or >70%, preferably: >75%
3h: >60%, or >65%, preferably: >85%, particularly >90%.

The extended release formulations in accordance with the present invention typically release 50% of the 40-O-(2-hydroxy)ethyl-rapamycin not earlier than 45, 60, 75, 90, 105 min or 120 min in said in-vitro dissolution assay.

In one preferred embodiment, the stable extended release formulations comprise 40-O-(2-hydroxy)ethyl-rapamycin in a fast dissolving or disintegrating carrier matrix in combination with coatings wherein at least one of the coatings is an extended release coating.

In another preferred embodiment, the stable extended release formulations comprise 40-O-(2-hydroxy)ethyl-rapamycin in a non-disintegrating carrier matrix with extended release properties, which can be combined optionally with additional coatings.

The carrier matrix comprises of matrix formers, typically matrix forming polymers, and may contain additional excipients, such as fillers, e.g. lactose, mannitol,maltodextrine, pregelatinized starch, calcium phosphate, or microcrystallline cellulose, and disintegrants, e.g. corn starch, croscamellose, sodium starch glycolate, or crospovidone, antioxidants, e.g. butylhydroxy anisol, butylhydroxy toluol, ascorbyl palmitate, tocopherol, vitamin E polyethylene glycol succinate, and process enhancing agents, such as lubricants and glidants, e.g. colloidal silicon dioxide, talc, glyceryl monostearate, magnesium stearate, calcium stearate, or sodium stearyl fumarate. The term "matrix former" typically relates to a pharmaceutically inert material which provides physical stability, such as e.g. mechanical or binding stability.

Suitable matrix forming polymers used for fast dissolving or disintegrating carrier matrices are known in the art include for instance cellulose or starch, for instance micro-crystalline cellulose ("MCC"), for example Avicel PH 101 (FMC BioPolymer), acacia, sodium alginate, gelatine, starch, pregeliatinised starch, methylcellulose, hydroxypropyl methylcellulose ("HPMC"), hydroxypropylcellulose, hydroxyethylcellulose, polyethylene glycol or polyvinylpyrrolidone ("PVP"), carrageenan, such as Gelcarin GP 812 or combinations thereof.

Suitable matrix forming excipients for non-disintegrating carrier matrices with extended release properties are known in the art include for instance acacia, sodium alginate, gelatine, carboxmethylcellulose sodium, (or "CMC sodium"), methylcellulose, ethylcellulose and cellulose acetate or polyacrylates, e.g. ammonio methacrylate copolymers (Eudragit RS/RL), hydroxypropyl methylcellulose ("HPMC"), hydroxypropylcellulose, hydroxyethylcellulose, polyvinylacetate, polyethylene glycol or polyvinylpyrrolidone ("PVP"), e.g. carrageenan, such as Gelcarin GP 812, glyceryl monostearate, stearylalcohol, stearic acid, glyceryl behenate, Vitamin E polyethylen glycol succinate, or combinations thereof.

In one embodiment, the extended release coating is a layer formed with water insoluble, non-disintegrating polymers, controlling the release by permeation of the drug through this layer. The extended release coating may also contain pore formers, plasticizers, and processing enhancing agents, such as lubricants and anti tacking agents.

Suitable extended release coating forming polymers which enable diffusion controlled release are known in the art include for instance ethylcellulose and cellulose acetate or polyacrylates, e.g. ammonio methacrylate copolymers (Eudragit RS/RL), polyvinylacetate or combinations thereof. In a preferred embodiment, the extended release coating forming polymer is ethylcellulose or cellulose acetate or polyacrylates, e.g. ammoniomethacrylate copolymer Type A (Eudragit RS) or ammonio-methacrylate copolymer Type B (Eudragit RL) or combinations thereof. Moreover, the extended release coating includes plasticizer, such as triacetine, triethyl citrate, dibutylsebacate, diethylsebacate, polyethylene glycol 3000, 4000 or 6000, acetyltriethylcitrate, acetyltributylcitrate, or diethylphthalate, and/or antitacking agents such Syloid 244 FP, talc, glyceryl monostearate, or titanium dioxide. The amount of plasticizer is typically between 5 to 40%, preferably 10 to 25%, relative to the amount of sustained release polymer.

The extended release coating is, in accordance with one preferred embodiment of the present invention, a pore forming system which comprises a water insoluble coating forming polymer and a pore former. The term "pore former" relates to a readily soluble excipient which allows pores to be introduced or permeability of the coating to be increased, and a diffusion controlled release of the active ingredient.

Suitable pore formers are known in the art include for instance hydroxypropylcellulose (HPC (e.g. Klucel™ EF, EXF,LF), or hydroxypropyl methylcellulose (HPMC, e.g. Methocel™ E3/E5, Pharmacoat 603™), polyethylen glycol (e.g. Macrogol 1500, 3500, 4000, 6000), poloxamer 188 (Pluronic F68™) or povidone (PVP, e.g. Kollidon K25/K30), a saccharide, e.g. a monosaccharide, such as dextrose, mannose, fructose, a disaccharide, such as sucrose or glucodifructose or combinations thereof. Preferably the pore former is hydroxypropylcellulose (HPC (Klucel™ EF, EXF,LF), or hydroxypropyl methylcellulose (HPMC, Methocel™ E3/E5, Pharmacoat 603™), polyethylen glycol (Macrogol 1500, 3500, 4000, 6000), poloxamer 188 (Pluronic F68™) or povidone (PVP, Kollidon K25/K30) or combinations thereof. Suitable amounts of pore formers included in coating are equal to ratios of coating polymer to pore former of e.g. 100:20 to 100:50, or 100:20 to 100:100, preferably ratios of 100:35 to 100:45, particularly ratios of 100:35 to 100:50 relative to the amount of coating forming polymer. Suitable amounts of coating forming polymers included are equal to percentages of polymer weight increase of e.g. 4% to 15%, 5% to 15%, preferably 5% to 12%, more preferably 6% to 12% weight of total weight of pharmceutical formulation.

In accordance with another preferred embodiment, the non-disintegrating extended release carrier matrix comprises matrix forming polymers which enable diffusion controlled release of the active ingredient by hydration of the polymer. The extended carrier matrix may contain further excipients, such as binders and or fillers and process enhancing agents, such as lubricants and glidants, etc.

Following matrix forming polymers are typically used for diffusion controlled release: sodium alginate, polyacrylic acids (or "carbomers"), carboxmethylcellulose sodium, (or "CMC sodium"), methylcellulose, ethylcellulose and cellulose acetate or polyacrylates, e.g. ammonio methacrylate copolymers (Eudragit RS/RL), hydroxypropyl methylcellulose ("HPMC") of different viscosity grades (i.e.average polymer chain lengths) and combinations thereof, e.g. Methocel™ CR grades, hydroxypropyl cellulose, e.g. Klucel™ HF/MF, polyoxyethylene, e.g. Polyox™ or polyvinylpyrrolidone ("PVP"), e.g. PVP K60, K90, carrageenan, such as Viscarin™ GP-209/GP-379, or combinations thereof. Combining of matrix forming polymers allows adjusting the dissolution rate of the active ingredient according to the need.

Alternatively, the non-disintegrating extended release matrix is formed with excipients, which enable release of the active ingredient by a controlled erosion. The erosion controlled matrices may contain lipophilic matrix formers, and also further excipients, such as fillers, disintegrants and process enhancing agents, such as lubricants and glidants.

Lipophilic matrix forming excipients related to this matrix type include lipophilic excipients, such as glyceryl monostearate, e.g. Cutina GMS, glyceryl behenate, e.g. Compritol 888 ATO, stearyl alcohol, stearic acid, hart fat, e.g. Gelucire™, or Vitamin E polyethylen glycol succinate, e.g. Speziol TPGS or combinations thereof.

Suitable binders, fillers or further excipients include for instance mannitol, pregelatinized starch, microcrystalline cellulose, lactose, calcium phosphate, talc, titanum dioxide, triethylcitrate, Aerosil, antioxidants such as e.g. BHT, desiccants and disintegrant such as e.g. crospovidone or sodium starch glycolate, starch, or croscarmellose.

In one preferred embodiment, the stable extended release formulations comprise 40-O-(2-hydroxy)ethyl-rapamycin in a fast dissolving/disintegrating matrix, e.g. in form of a solid dispersion as described hereinbelow, in combination with functional layers or coatings wherein at least one of the functional layer(s) or coating(s) has release controlling behavior enabling extended release of the active ingredient. In another preferred embodiment, the stable extended release formulations comprise 40-O-(2-hydroxy)ethyl-rapamycin in the extended release matrix which, optionally, can further contain functional layers or coatings, such as protective or sustained release layers or coatings. The coatings, e.g. the extended release coating, typically has a coating thickness in the range of 10 to 100 µm, preferably 10 to 50 µm (assessed by confocal RAMAN spectroscopy).

In one preferred embodiment of the present invention, the formulations of the present inventions are in form of a multiparticulate delivery system. Multi-particulate drug delivery systems in accordance with the present invention are mainly oral dosage forms consisting of multiple, small discrete dose units. In these systems, the dosage form, of the drug substances such as capsule, tablets, sachet or stickpack, contains a plurality of subunits, typically consisting of tens to hundreds or even up to thousands of spherical particles with diameter of 0.05-2.00mm. Formulations of the size 1.5 - 3 mm, e.g. minitablets, present another alternative of the present invention. The dosage form is designed to disintegrate rapidly in the stomach releasing the multiparticulates. The multiparticulates are spread in the gastro-intestinal lumen and will be emptied gradually from the stomach releasing the drug substance in a controlled manner.

In one embodiment the pharmaceutical compositions according to the present invention, e.g. in form of multi-particulate delivery system, comprise O-(2-hydroxy)ethyl-rapamycin as active ingredient, e.g. dissolved or dispersed in the core of the particle, (e.g. a bead, pellet, granule or minitablet), or in a layer surrounding an inert core of the particle. The active ingredient can be for instance be embedded in an extended release matrix, preferably comprising a hydrophilic or lipophilic matrix forming excipients, or embedded in a fast disintegrating and/or dissolving matrix in combination with functional layer(s) and top coating(s) wherein at least one of the functional layer(s) or top coating(s) comprises a coating forming polymer enabling diffusion controlled extended release of the active ingredient. Optionally, a protection layer for improving stability of the active ingredient separates the matrix containing the active substance from functional layers or top coatings, to ensure stability of the drug product.

In a another preferred embodiment, the present invention provides stable extended release formulations, e.g. in form of a multiparticulate delivery system, comprising 40-O-(2-hydroxy)ethyl-rapamycin as active ingredient and an outer coating layer comprising an insoluble polymer and a soluble component as pore former, and optionally further functional layers. For the purpose of the present invention the terms "outer layer" is a layer located towards to the outside of a particle and may be coated with a further layer(s) or may be a top coating. The terms "outer layer", "coating layer" or "top coat" may be used interchangeably depending on the context in which the terms are used.

In a preferred embodiment, the pharmaceutical compositions of the present invention contain 40-O-(2-hydroxy)ethyl-rapamycin as sole therapeutically active ingredient.

In one preferred embodiment, the particles comprise one or several top coats enabling extended release of the active ingredient. Top coats typically are final layers with release controlling behaviour, which are enclosing each particle of the multiparticulates separately.

In a particularly preferred embodiment, the pharmaceutical compositions of the present invention comprise an outer layer or a top coating that controls the release by the diffusion of the drug through the coating layer which is permeable, optionally by the formation of pores in the insoluble polymer layer, or alternatively solely by the hydration of the insoluble polymer, or that controls the release by a combination of a pore former and hydration of the insoluble polymer. The polymer is insoluble independently from pH, and optionally contains water soluble pore former. The release rate is affected by the extent of pore formation after the pore former is dissolved. The insoluble coating polymer can be cellulose ethers such as ethylcellulose and cellulose acetate or polyacrylates, e.g. ammonio methacrylate copolymers (Eudragit RS/RL). Suitable pore formers include water soluble cellulose ethers, for instance hydroxypropylcellulose (HPC (Klucel™ EF, EXF, LF) or hydroxypropyl methylcellulose (HPMC, Methocel™ E3/E5, Pharmacoat 603™), polyethylen glycol (Macrogol 1500, 3500, 4000, 6000), poloxamer 188 (Pluronic F68™) or povidone (PVP, Kollidon K12, K25, K30). For instance, water soluble pore former can be mixed with insoluble polymer in a ratio of 2:1 to 1:10, e.g. 1:1 to 1:5, 1:3 or 1:5. A preferred pore former to insoluble polymer ratio in accordance with the present invention is HPC, e.g Klucel™ EF,EXF,LF or HMPC 3cP, e.g. Methocel™ E3, in a ratio of 1:1 to 1:4, e.g. about 1:1, 1:1.2, 1:1.5 or 1:2. The preferred insoluble polymers in accordance with the present invention are ethylcellulose (EC, Aqualon EC N10™) in combination with a pore former. Without the use of a pore former, preferably the combination of the insoluble polymers ammoniomethacrylate copolymer Type A (Eudragit RS) and ammonio-methacrylate copolymer Type B (Eudragit RL) at ratios of 1:2 to 9:1, preferably 1:1 to 4:1 are applied in accordance with this invention.

The sustained release top coat(s) preferably achieve release of majority of the active substance into the small intestine and allows to protect the active substance from stomach fluids and minimizes the exposure of the active substance to the mouth, esophagus and stomach.

In one embodiment of the present inventions, the pharmaceutical compositions of the present invention comprise a drug substance containing matrix, e.g. fast disintegrating and/or dissolving matrix layer or in an extended release matrix layer, e.g. on a starter core such as beads, pellets or granules, which can consist of one or more components, and in which the active ingredient is dispersed or dissolved. For instance, amorphous or crystalline 40-O-(2-hydroxy)ethyl-rapamycin can be dispersed or dissolved in the matrix in a ratio from 1:100 to 100:1 in the matrix. In a particularly preferred embodiment the 40-O-(2-hydroxy)ethyl-rapamycin ratio to matrix former is 1:50 to 5:1; or 1:50 to 1:1 by weight, or more preferred 1:5 to 2:3, or yet more preferred 1:10 to 1:5 by weight (as to the matrix former).

According to one embodiment of the present invention, the drug substance containing matrix is layered onto the surface of starter cores. The layer is built by spraying a dispersion or solution of the matrix components and the drug substance on to particles of uniform, regular size and shape in a fluid bed process. Alternatively, powder mixtures of the matrix components can be layered using a rotating disk processor. Starter cores have an average particle size 0.1 to 2.5 mm. They can be single crystals, e.g. sucrose, or granular agglomerates manufactured by fluid bed granulation, a rotorgranulation, extrusion and spheronization, or a compaction process. This encompasses also minitablets that can be used as starter cores. Preferably, the starter cores have a spherical shape and consist of inert material such as sucrose and starch (Sugar Spheres, Suglets™, Non-pareils), mannitol (e.g. MCells™), lactose (e.g. spray dried lactose) or microcrystalline cellulose (e.g. Cellets™).

In another embodiment of the invention, the drug substance containing matrix is incorporated in the cores of the particles. The matrix forming excipients, fillers, and other ingredients for enhancing the process are mixed together with the drug substance. The powder mixtures obtained can be formulated as particles by using wet extrusion or melt extrusion and subsequent spheronization, or by compacting the mixtures to minitablets. The matrices formed could be either fast disintegrating/dissolving matrices, or non-disintegrating matrices with extended release properties built with hydrophilic or lipophilic matrix forming excipients. In a one embodiment, multiparticulates consisting of a hydrophilic, non-disintegrating matrix which contains the drug substance or a solid dispersion thereof, are prepared by mixing the active ingredient, a filler, e.g. lactose, together with hydrophilic, hydrogel forming polymers with different viscosities, a glidant, and a lubricant. The hydrophilic, hydrogel forming polymer is preferrrably for example hydroxypropyl methylcellulose, with low viscosity grade of less than 20 mPas for a 2% by weight aqueous solution, e.g. Methocel E5, combined with hydroxypropyl methylcellulose grade with high viscosity of more than 100 mPas for a 2% by weight aqueous solution, e.g. Methocel K100. The powder mixture is then compressed on the tabletting machine to obtain minitablets. Alternatively, the powder mixture can be wetted with organic solvente, e.g. ethanol, and then extruded and spheronized for obtaining multiparticulartes.

In another embodiment, multiparticulates consisting of a lipophilic, non-disintegrating matrix which contains the drug substance or a solid dispersion thereof are prepared by mixing the active ingredient, lipophilic, meltable, matrix forming excipients, and fillers. The mixture is processed by melting and mixing in an extruder. The obtained extudate strands are cut into particles and are optionally spheronized. The lipophilic excipients used are for example Vitamin E polyethylen glycol succinate (Vit E TPGS, e.g. Kolliphor TPGS Pharma from BASF) solely, or in combination with glycerol monostearate (GMS, e.g. Kolliwax GMS fromBASF) at ratios of 9:1 to 1:9.

The pharmaceutical compositions according to the present invention have been found to reduce the peak concentration (Cₘₐₓ) to concentration at 24 hours post-dose (C₂₄ₕ) ratio after a single dose administration in 24 healthy subjects, as compared to the current 40-O-(2-hydroxy)ethyl-rapamycin tablets available to patients (Final Market Image or "FMI" tablets). A typical Cₘₐₓ of the formulations according to the present invention is <10ng/ml. The reduced Cₘₐₓ/C₂₄ₕ ratio, by pharmacokinetic model simulations, is predicted to reduce the Cₘₐₓ to minimum concentration (Cₘᵢₙ) ratio (Cₘₐₓ/Cₘᵢₙ) in a concentration-time profile during a 24-hour dosing interval after daily administration of the present invention. The advantage of the reduced Cₘₐₓ/Cₘᵢₙ ratio of the present invention is that, with the appropriate dose based on the bioavailability of the present invention relative to the FMI formulation, the present invention enables the concentration of everolimus to maintain above the lower therapeutic range of everolimus (for sufficient efficacy) and at the same time distance away from the upper therapeutic range of everolimus (concentration region of toxicity). Thus, the present invention is able to improve the safety profile of everolimus without affecting its efficacy. The pharmaceutical compositions according to the present invention thus allow for instance better exploitation of the therapeutic window of 40-O-(2-hydroxy)ethyl-rapamycin. Typical Cₘₐₓ/C₂₄ₕ (thus typical Cₘₐₓ/Cₘᵢₙ) ratio in patients having administered the pharmaceutical compositions according to the present inventions is < 5 or <4, e.g. 3.5±1 or 3±0.5.

In accordance with one embodiment of the present invention, 40-O-(2-hydroxy)ethyl-rapamycin is contained in a layer separate from the functional layer or top coat controlling the extended release properties of the formulation. Such layer may be made of any substance which is suitable for dispersing or dissolving O-(2-hydroxy)ethyl-rapamycin. In a preferred embodiment, the layer comprising O-(2-hydroxy)ethyl-rapamycin is made of a hydrophilic carrier matrix. The carrier matrix is embedding the active ingredient and protecting it thereby against degradation. Suitable matrix formers are hydrophilic polymers, e.g. HPMC type 2910 or type 2280, HPC, HEC, MEC, MHEC, povidone, which can be dissolved or rapidly dispersed in water. In one preferred embodiment, the matrix layer is in form of a solid dispersion, for instance as described in WO97/03654 or WO03/028705.

In a preferred embodiment, the fast dissolving/disintegrating carrier matrix for 40-O-(2-hydroxy)ethyl-rapamycin is in form of a solid dispersion. The solid dispersion for instance comprises a carrier, e.g. a water-soluble polymer, for example one or a mixture of the following polymers may be used:
- hydroxypropylmethylcellulose (HPMC), e.g. Hypromellose type 2910, which is available as Methocel™ E from Dow Chemicals or Pharmacoat™ from Shin Etsu. Good results may be obtained using HPMC with a low apparent viscosity, e.g. below 100 cps as measured at 20°C for a 2% by weight aqueous solution, e.g. below 50 cps, preferably below 20 cps, for example HPMC 3 cps;
- polyvinylpyrrolidone (povidone, PVP), e.g. PVP K25, K30 or PVP K12. PVP is available commercially, for example, as Kollidon® from the BASF company or as Plasdone® from ISP company. A PVP having an average molecular weight between about 8,000 and about 50,000 Daltons is preferred, e.g. PVP K30;
- hydroxypropylcellulose (HPC), e.g. Klucel EF/LF/JFor a derivative thereof. Examples of HPC derivatives include those having low dynamic viscosity in aqueous media, e.g. water, e.g. below about 400 cps as measured in a 5 % aqueous solution at 25°C. Preferred HPC derivatives an average molecular weight below about 200,000 Daltons, e.g. between 80,000 and 140,000 Daltons. Examples of HPC available commercially include Klucel® LF, Klucel® EF and Klucel® JF from the Hercules Aqualon company; and Nisso® HPC-L available from Nippon Soda Ltd;
- a polyethylene glycol (PEG). Examples include PEGs having an average molecular weight between 1000 and 9000 Daltons, e.g. between about 1800 and 7000, for example PEG 2000, PEG 4000, or PEG 6000 (Handbook of Pharmaceutical Excipients, p. 355-361);
- a saturated polyglycolised glyceride, available for example, as Gelucire®, e.g. Gelucire® 44/14, 53/10, 50/13, 42/12, or 35/10 from the Gattefossé company; or
- a cyclodextrin, for example a β-cyclodextrin or an α-cyclodextrin. Examples of suitable β-cyclodextrins include methyl-β-cyclodextrin; dimethyl-β-cyclodextrin; hydroxyproypl-β-cyclodextrin; glycosyl-β-cyclodextrin; maltosyl-β-cyclodextrin; sulfo-β-cyclodextrin; a sulfo-alkylethers of β-cyclodextrin, e.g. sulfo-C₁₋₄-alkyl ethers. Examples of α-cyclodextrins include glucosyl-α-cyclodextrin and maltosyl-α-cyclodextrin.

In one preferred embodiment, the O-(2-hydroxy)ethyl-rapamycin-containing layer, contains antioxidant in a ratio of 1:1000 to 1:1 related to the amount of drug substance. The antioxidant may also be present in other functional layers, e.g. at concentration of 0.1 to 10%, preferably 0.1 to 1%. Suitable antioxdants include for instance butyl hydroxyl toluol, butyl hydroxy anisol, ascorbyl palmitate, tocopherol, vitamin E polyethylene glycol succinate. In a preferred embodiment, the antioxidant is butyl hydroxyl toluol.

In one preferred embodiment, a protection layer separates the layer containing the active substance from other functional layers, such as e.g. the top coating, to enhance stability of the of the drug product. The drug substance is stabilized by excluding any direct contact with the top coating. The protection layer also acts as diffusion barrier preventing any components in the top coating, e.g. polymer by-products or plasticizers, which can migrate through the layers, from getting in direct contact with the active. Beside the polymers, which are used also as matrix formers (e.g. the matrix formers described above), high content, of inorganic pigments or antitacking agents such as talc and/or titanium dioxide, e.g. 10 to 100%, preferreable 20 to 50%, relative to the applied amount of polymer, contribute to the barrier function. The protection layer thickness can be adjusted to gain optimized drug product stability.

In another preferred embodiment, the active ingredient 40-O-(2-hydroxy)ethyl-rapamycin is directly embedded in the extended release carrier matrix as herein described.

The pharmaceutical compositions of the present invention provide good stability for active substance such as e.g. 40-O-(2-hydroxy)ethyl-rapamycin.

In accordance with a further aspect of the present invention, the present invention contains strongly hygroscopic excipients, which are able to bind water moisture enclosed in the formulation working as an internal desiccant. Adsorbents such as e.g. crospovidone, croscarmellose sodium, sodium starch glycolate, or starch can be used.

In a preferred embodiment methods to stabilize 40-O-(2-hydroxy)ethyl-rapamycin using crospovidone are provided. Crospovidone is known and widely used as tablet disintegrant. It has surprisingly been found in accordance with the present invention that crospovidone protects 40-O-(2-hydroxy)ethyl-rapamycin from moisture induced degradation. Thus, the present invention provides a method to reduce or prevent moisture induced degradation of 40-O-(2-hydroxy)ethyl-rapamycin using 2% to 25% crospovidone. The crospovidone is part of the powder mixtures used for wet and melt extrusion, part of the powder blend for compressing the minitablets, part of powder blend for tabletting the multiparticulates, are directly added to the multiparticulates in a sachet or capsule filling process. In a related embodiment, the present invention provides the use of crospovidone as internal desiccant for pharmaceutical formulations comprising 40-O-(2-hydroxy)ethyl-rapamycin.

In one aspect, the present invention provides O-(2-hydroxy)ethyl-rapamycin containing particles (0.1 to 0.5 mm), beads, pellets (0.2 to 2 mm) or mini-tablets (1.5 to 3 mm), with a low water moisture content of less than 5% in total or even more preferred with less than 3% or less than 2.5% in total.

In another aspect, the present invention contains strongly hygroscopic excipients which are able to bind water moisture enclosed in the formulation working as an internal desiccant. Adsorbents such as e.g. crospovidone, croscarmellose sodium, sodium starch glycolate, starch can be used.

A common side effect O-(2-hydroxy)ethyl-rapamycin formulations is mucositis, which can lead to additional suffering of the patients, poor patient compliance and suboptimal efficacy. The underlying cause for mucositis is not known and could for instance be due to local irritation of the mucous membranes, but also due to a systemic effects. The formulation of the present invention can reduce or eliminate mucositis as side effect of O-(2-hydroxy)ethyl-rapamycin administration.

The pharmaceutical compositions, e.g. a multiparticulate delivery system of according to the present invention can be formulated into a drug product such as e.g. capsules (e.g. HPMC or Hart Gelatine capsules), or filled into sachets or stick-packs, or formulated as tablets which release the particles upon disintegration.

For further improvement of the drug product stability, the primary packaging, such as sachets, stickpacks, blisters or bottles may include an water sorbing ingredient, eg.e silica gel, which is reducing or stabilizing the water moisture content of the drug product during shelf life storage and/or in during in-use time.

The formulation of the present invention may consist of and/or release multiple pellets, granules or minitablets.

Where the pharmaceutical composition of this invention is in form of a dosage unit, e.g. as a tablet, capsule, granules, each unit dosage will suitably contain between 0.1 mg and 40 mg of the drug substance, more preferably between 1 and 20 mg; for example 0.1, 0.25, 0.5, 0.75, 1.0, 2.0, 2.5, 3.0, 5.0, 10 and 20 mg. Further suitable dosage units include e.g. 25 mg or 30mg or 35 mg or 40 mg or 50 mg. Such dosage units are suitable for administration 1 to 5 times daily depending upon the particular purpose of therapy, the phase of therapy and the like. In one embodiment the unit dosage form is administered once daily. The exact amount of the compositions to be administered depends on several factors, for example the desired duration of treatment and the rate of release of O-(2-hydroxy)ethyl-rapamycin.

The formulations of the present invention have further advantageous properties over currently used formulations. For instance, the formulations of the present invention:
- allow flexible dose adjustments
- allow to meet a tailored drug release profile, e.g. by combining granules, beads, pellets or minitablets with different release profiles (e.g. an initial pulse and sustained release)
- allow to prevent contact of drugs with mucus membrane in the mouth
- allow extended release coated pellets, granules or mini-tablets protect the drug in the stomach against degradation leading to higher bioavailability
- allow extended release profiles
- protect the stomach mucosa against irritation through direct contact with the drug
- lower Cmax and reduce Cmax/Cmin ratio
- reduce inter and/or intra-patient variability in Cmax and AUC
- reduce food dependent inter- and/or intra-patient variability in Cmax and AUC.

It has been found in accordance with one embodiment of the present invention that the pharmaceutical compositions of the present invention allow administering a higher dose of O-(2-hydroxy)ethyl-rapamycin compared to the immediate release O-(2-hydroxy)ethyl-rapamycin formulations available on the market, but at the same time having an improved safety profile. Accordingly, in one embodiment, the present invention provides an extended release pharmaceutical formulation or a solid dosage form for use as a medicine. In another embodiment the present invention provides a method for the treatment of mTOR sensitive diseases e.g. as described hereinbelow wherein O-(2-hydroxy)ethyl-rapamycin is administered as 15mg, 20mg, 25mg, 30mg, 35mg, 40mg, 45mg or 50mg dose, e.g. once per day. In a preferred embodiment O-(2-hydroxy)ethyl-rapamycin is administered is administered 1 mg to 40mg, e.g. 20mg to 40mg (e.g. 20mg, 25mg, 30mg, 35mg or 40mg) once per day or 2mg to 80mg, e.g. 20mg to 80mg (e.g. 20mg, 30mg, 40mg, 50mg, 60mg, 70mg or 80mg) every second day or 5mg to 150mg, e.g. 40mg to 150mg (e.g. 40mg, 50mg, 60mg, 80mg, 100mg, 120mg or 150mg) once per week. mTOR sensitive diseases include in particular solid tumor diseases, e.g. renal cell carcinoma, TSC, gastric cancer, breast cancer, lymphoma, hepatocellular cancer.

The drug pharmaceutical compositions according to the present inventions, e.g. multiprticulates formulations, can be prepared either by extruding and spheronizing a mixture of the matrix forming excipients together with the drug substance with the aid of heat or wetting liquids, or by compacting minitablets with drug containing mixtures, or by layering the drug containing matrix layer onto cores in a fluid bed or rotogranulation process.

The layer containing the active substance can be prepared by spraying a spray dispersion with organic solvents in which the hydrophilic components and the active substance are dispersed or dissolved, preferably dissolved, onto the core material, while concurrently the solvents are continuously removed by the aid of heated, dry air. By this process a matrix layer surrounding the cores is formed, more preferably the layer formed is a solid dispersion of the active in polymers such as e.g. HPMC, HPC, HEC.

Pharmaceutical formulation according to the present inventions can for instance be prepared as follows: An organic feed mixture for spraying in which the hydrophilic polymer is dispersed in colloidal manner and 40-O-(2-hydroxy)ethyl-rapamycin is dispersed or dissolved, which precipitate together as a uniform, smooth layer of solid dispersion upon removal of the solvent in such a way that they for instance can be coated with modified release coats.

The obtained drug containing multiparticulates can be coated with additional functional layers and top coatings. A spray dispersion containing coating polymers, lubricants, anti tack agents, pore formers and plastisizers, which are dissolved, dispersed and suspended in organic solvents and mixtures thereof, is sprayed onto the drug containing multiparticulates. During processing the multiparticulates are kept continuously in a controlled motion or fluidization, while dry, heated process gas is applied to the product bed for evaporating the solvents from the surface of the multiparticulates, where the film layer is formed at a defined temperature. The film layer thickness can be controlled by the amount of coating dispersion sprayed. Final drying is applied for minmizing the residual solvent content in the layered and coated multiparticulates.

The multiparticulates can be filled into hard capsules, into sachet, stickpacks, or compressed into tablets after mixing them with suitable tabletting agents.

Also provided are treatment methods for mTOR pathway senstive diseases, such as e.g. described below, by using pharmaceutical composition according to the present invention, e.g. a multiparticulate delivery system.

The oral pharmaceutical compositions of this invention are useful for the treatment or prevention of diseases or conditions responsive to inhibition of mTOR signaling pathway e.g. the following conditions:
a) Treatment and prevention of organ or tissue allo- or xeno-transplant rejection, e.g. for the treatment of recipients of e.g. heart, lung, combined heart-lung, liver, kidney, pancreatic, skin or corneal transplants. They are also indicated for the prevention of graft-versus-host disease, such as following bone marrow transplantation.
b) Treatment and prevention of autoimmune disease and of inflammatory conditions, in particular inflammatory conditions with an etiology including an autoimmune component such as arthritis (for example rheumatoid arthritis, arthritis chronica progrediente and arthritis deformans) and rheumatic diseases. Specific autoimmune diseases for which the compounds of the invention may be employed include, autoimmune hematological disorders (including e.g. hemolytic anaemia, aplastic anaemia, pure red cell anaemia and idiopathic thrombocytopenia), systemic lupus erythematosus, polychondritis, sclerodoma, Wegener granulamatosis, dermatomyositis, chronic active hepatitis, myasthenia gravis, psoriasis, Steven-Johnson syndrome, idiopathic sprue, autoimmune inflammatory bowel disease (including e.g. ulcerative colitis and Crohn's disease) endocrine ophthalmopathy, Graves disease, sarcoidosis, multiple sclerosis, primary billiary cirrhosis, juvenile diabetes (diabetes mellitus type I), uveitis (anterior and posterior), keratoconjunctivitis sicca and vernal keratoconjunctivitis, interstitial lung fibrosis, psoriatic arthritis, glomerulonephritis (with and without nephrotic syndrome, e.g. including idiopathic nephrotic syndrome or minimal change nephropathy) and juvenile ermatomyositis.
c) Treatment and prevention of asthma.
d) Treatment of multi-drug resistance (MDR). MDR is particularly problematic in cancer patients and AIDS patients who will not respond to conventional chemotherapy because the medication is pumped out of the cells by Pgp. The compositions are therefore useful for enhancing the efficacy of other chemotherapeutic agents in the treatment and control of multi drug resistant conditions such as multidrug resistant cancer or multi drug resistant AIDS.
e) Treatment of proliferative disorders, e.g. tumors, hyperproliferative skin disorder and the like for instance solid tumors: e.g. Renal Cell Carcinoma, Neuroendocrine tumor e.g. GEP Neuroendocrine Tumors, Phaeochromocytoma, Meningioma, Head and neck squamous cell carcinoma, Breast Cancer, Lymphoma NOS, Thyroid carcinoma NOS Endometrial cancer, Hepatocellular carcinoma, Prostatic Cancer, Metastatic melanoma, Glioma, Glioblastoma Multiforme, Non-small cell Lung Cancer (NSCLC), Mastocytosis, Metastatic Lung Cancer, Hepatocellular carcinoma, Gastrointestinal Stromal Tumours (GIST), Hepatocellular carcinoma, Astrocytoma, Tuberous sclerosis, e.g. SEGA, AML, Lymphangioleiomyomatosis, Thyroid carcinoma NOS, Bile duct Cancer, colorectal Cancer, Adenoid cystic carcinoma, Cholangiocarcinoma, Sarcoma NOS, Mesothelioma, Malignant hepatic neoplasm, colorectal Cancer, Metastatic melanoma, Cervical Cancer, Metastatic Breast Cancer, Bladder Cancer, Non-Hodgkin's lymphoma, Hodgkin's lymphoma, Kaposi's sarcome, Squamous cell carcinoma, Urothelial Cancer, Neoplasm of Digestive Organs, Gastric Cancer, pancreatic Cancer; or liquid tumors: e.g. Advance Hematologic Malignancies, e.g. Leukaemia, e.g. acute myeloid leukemia, Acute Myeloid Leukaemia, Multiple myeloma. f) Treatment of abnormally increased bone turnover or resorption, e.g. osteoporosis, bone loss associated e.g. with aromatase inhibitor treatment, rheumatoid arthritis, osteopenia, osteogenesis imperfecta, hyperthyroidism, anorexia nervosa, organ transplantation, joint prosthesis loosening, periarticular bone erosions in rheumatoid arthritis, osteoarthritis, hypercalcemia, bone cancer and bone metastases induced by a primary tumour, multiple myeloma.
f) Treatment of fungal infections.
g) Treatment and prevention of inflammation, especially in potentiating the action of steroids.
h) Treatment and prevention of infection, especially infection by pathogens having Mip or Mip-like factors.
i) Treatment of overdoses of FK-506 and other macrophilin binding immunosuppressants.

The following Examples illustrate the invention described above; they are not, however, intended to limit the scope of the invention in any way. The beneficial effects of the formulations of the invention can also be determined by other test models known as such to the person skilled in the pertinent art.

Exemplified below are some examples of pharmaceutical formulations comprising 40-O-(2-hydroxy)ethyl-rapamycin that, when administered, lead to reduced average plasma peak concentrations, reduced inter- and intra-patient variability in the extent of drug absorption and in the plasma peak concentration, reduced Cmax / Cmin ratio and show reduced food effect. The formulations are more robust, more stable and safer. In addition, the composition of the formulation or the process for preparing the formulation allows that the desired release profile is more precisely reached.

### EXAMPLES

### Example 1:

### Protection layered pellets for a dose of 5 mg everolimus:

The following example of drug layered and protection layered pellets provide an immediate release form of mulitparticulates which can be further coated to receive a product with extended release properties. Drug load is adjusted to a percentage, which allow for filling of 5 mg into capsule size 0. According to the two different compositions described in table 1 different thicknesses of the protection layer can be realized to optimize protective effect.

A procedure for preparing multiparticulates with a drug containing matrix layer is as follows: The matrix forming polymer HPMC (type 2910, 3 cP) is dispersed in ethanol at a ratio of 4:1 related to the drug substance with a final concentration of 6% in the solvents. Antioxidant butyl hydroxy toluol is added to the dispersion at an amount equal to 2% related to drug substance. A small fraction of water equal to 6% of total amount of solvents is used for dispersing 7.5% talc and 3.0% titanium dioxide based on solids in the layer with the aid of a homogenizer. The aqueous suspension is added to the dispersion. During continuous stirring the dispersion is equilibrated until the swollen polymer particles will be disintegrated. Finally, the drug substance will be added and dispersed in the coating dispersion prior to starting the layering onto sugar spheres of 355 to 425 µm, preheated and fluidized in a fluid bed processor. The amount of sugar spheres used results in a drug concentration of 1.5% in the active layered multiparticulates after spraying. The spraying occurs at a controlled product bed temperature in the range between 35° and 45°C using a tangential spray process. After finishing ther spraying process, when a weight gain of 9.2% is received, the obtained multiparticulates will be dried in the fluid bed at temperatures up to 65°C.

A subsequent layering procedure follows for applying a protective, stability enhancing layer: The binding polymer HPMC (type 2910, 3 cP) is dispersed in ethanol with a final concentration of 4% in the solvents. A small fraction of water equal to 6% of total amount of solvents is used for dispersing 25% talc and 5% titanium dioxide with the aid of a homogenizer. The aqueous suspension is added to the dispersion. During continuous stirring the dispersion is equilibrated until the swollen polymer particles will be disintegrated. The active layererd multiparticulates are preheated and fluidized in a fluid bed processor. The spraying is conducted at a controlled product bed temperature in the range between 35° and 45°C using a bottom spray process until a weight gain of 10 to 15% is received. After finishing ther spraying process, the obtained multiparticulates will be dried in the fluid bed at temperatures up to 65°C.

**Table 1**

| | | | | | |
|---|---|---|---|---|---|
| | | | | | |

| Protection layered pellets, 5mg everolimus | | | | | |
|---|---|---|---|---|---|
| | | With 15% weight gain protection layer | | With 10% weight gain protection layer | |
| Processing step | Ingredients | % | mg/unit | % | mg/unit |
| Active layering | Sugar spheres 355-425 µm | 76.64 | 305.29 | 82.6 | 277.52 |
| | Everolimus | 1.30 | 5.00 | 1.50 | 5.00 |
| | Butyl Hydroxy Toluol | 0.03 | 0.10 | 0.03 | 0.10 |
| | Hypromellose 2910 3 cP | 5.22 | 20.00 | 6.0 | 20.00 |
| | Titan Dioxide | 0.22 | 0.84 | 0.3 | 0.84 |
| | Talc | 0.55 | 2.10 | 0.6 | 2.10 |
| Protection layer coating | Hypromellose 29103cP | 10.03 | 38.46 | 7.0 | 23.51 |
| | Talc | 2.51 | 9.62 | 1.7 | 5.88 |
| | Titan Dioxide | 0.50 | 1.92 | 0.3 | 1.18 |
| Total: | | 100.00 | 383.33 | 100.00 | 336.12 |

### Example 2:

### Protection layered pellets for a dose of 20mg everolimus:

In this example another variant of pellets produced by layering and coating is provided. Immediate release pellets have higher drug load than in example 1 allowing for the manufacture of higher dose strengths. With this variant 10 or 20 mg can be filled into hard capsule of size 1. The material can be used of different kind of extended release coatings. Multiparticulates layered with a matrix containing the active and subsequently layered with a protective layer are produced as described in example 1. Deviant from example 1, the matrix forming polymer HPMC (type 2910, 3 cP) is dispersed in ethanol at a ratio of 3:2 related to the drug substance with a final concentration of 5% in the solvents. The concentration of active in the active layered pellets is increased form 1.5% in example 1 to 10%.

**Table 2:**

| Protection layered pellets, 20mg everolimus | | | |
|---|---|---|---|
| Processing step | Ingredients | % | mg/unit |
| Active layering | Sugar spheres 355-425 µm | 66.22 | 145.67 |
| | Everolimus | 9.09 | 20.00 |
| | Butyl Hydroxy Toluol | 0.18 | 0.40 |
| | Hypromellose 2910 3 cP | 13.57 | 29.85 |
| | Talc | 1.85 | 4.07 |
| Protection layer | Hypromellose 2910 3 cP | 6.99 | 15.38 |
| coating | Talc | 1.75 | 3.85 |
| | Titan Dioxide | 0.35 | 0.77 |
| Total: | | 100.00 | 220.00 |

### Example 3:

### Extended release pellets 5mg coated with Eudragit RS/RL

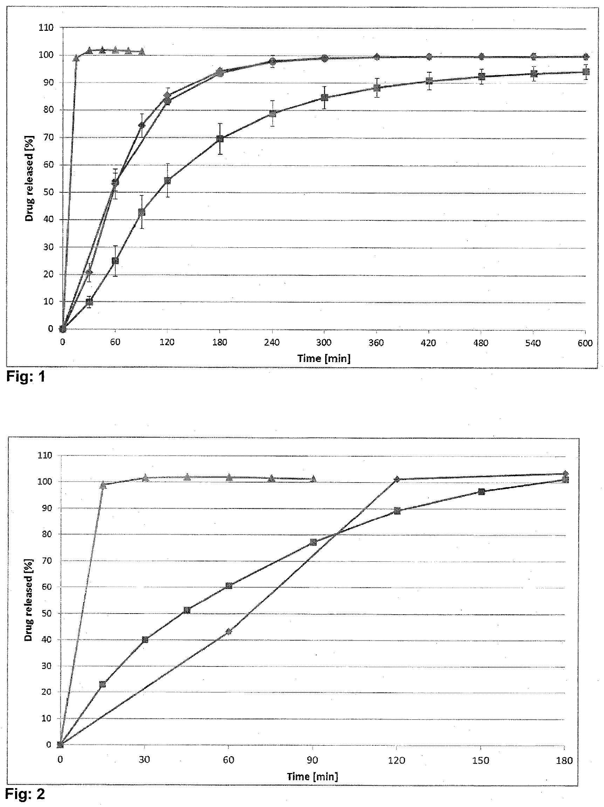

This example is providing a possibility how an extended release profile can be achieved by top coating of immediately release pellets such as pellets from table 3. A combination of insoluble polymers Eudragit RS and Eudragit RL can be properly adjusted to lead to a product with the desired release properties. In this case release was completed within 2 hours (see figure 2).

A coating is applied to the protective layered multiparticulates to obtain a product with sustained release properties:
Sustained release polymers Eudragit RL100 and Eudragit RS100 at a ratio of 3:7 are dissolved in acetone obtaining a final concentration of 14% in the solvents. While the solution is continuously stirred, 5% anti tack agent glyceryl monostearate and 10% plasticizer triethylcitrate are added and dissolved at an amount relative to the amount of ammonio methacrylicacid copolymer (Eudragit RS/RL). A small fraction of water equal to 5% of total amount of solvents is used for dispersing 30% talc with the aid of a homogenizer. The aqueous suspension is added to the polymer solution.

The protective layered multiparticulates are preheated and fluidized in a fluid bed processor prior to starting spraying the dispersion. The spraying is conducted at a controlled product bed temperature in the range between 35° and 45°C using a bottom spray process until a polymer weight gain of 14% is received. After finishing the spraying process, the obtained multiparticulates will be dried in the fluid bed at temperatures at 40°C for 15 min.

Finally the coated multiparticulates were filled manually into HPMC hard capsules of size 0. The fill weight was adjusted to amount equivalent to 5 mg everolimus.

**Table 3**

| Pro-cessing step | Ingredients | % | mg/unit |
|---|---|---|---|
| Active layering | Sugar spheres 355-425 µm | 83.3 | 305.29 |
| | Everolimus | 1.4 | 5.00 |
| | Butyl Hydroxy Toluol | 0.03 | 0.10 |
| | Hypromellose 2910 3 cP | 5.5 | 20.00 |
| | Titan Dioxide | 0.2 | 0.84 |
| | Talc | 0.6 | 2.10 |
| Protection layer coating | Hypromellose 2910 3 cP | 7.0 | 25.64 |
| | Talc | 1.7 | 6.41 |
| | Titan Dioxide | 0.3 | 1.28 |
| Total: | | 100.0 | 366.67 |

**Table 4:**

| Extended release coated multiparticulates Everolimus 5mg, Eudragit RS/RL 7:3, 16.9% polymer weight increase | | | |
|---|---|---|---|
| Processing step | Ingredients | % | mg/unit |
| Top coating | Protection layered pellets 5mg, Table1 | 80.2 | 366.67 |
| | Eudragit RS 100 | 9.5 | 43.00 |
| | Eudragit RL 100 | 4.1 | 19.00 |
| | Talc | 4.1 | 18.9 |
| | Glyceryl Monostearate | 0.7 | 3.15 |
| | Triethyl citrate | 1.4 | 6.30 |
| Total: | | 100.00 | 440.80 |

### In-vitro dissolution method:

The multiparticulates were filled into hard capsules of size 0 and then placed into a dissolution vessel filled with 900 mL phosphate buffer pH 6.8 containing sodium dodecyl sulfate 0.2% at 37°C. The dissolution was performed using a paddle method at 75 rpm according to USP monograph 711, and Ph.Eur. monograph 2.9.3., respectively.

### In-vitro dissolution results:

The release profile is shown in figure 2.

| Minutes | % released Table 4 |
|---|---|
| 60 | 43.2 |
| 120 | 101.3 |
| 180 | 103.5 |

### Example 4:

### Extended release pellets 5mg coated with Eudragit RURS

A very fast relasing coating was applied to proctection layered pellets with a drug load of 2.6% using only Eudragit RL100 as polymer. The coating spray fluid was prepared using a solvent mixture of isopropanol/acetone 60:40. The polymer concentration in the solvent was set to 10%(w/w). The polymer weight increase was 7.4%.

**Table 5**

| Protection layered pellets with 2.6% drug load Everolimus 5mg | | | |
|---|---|---|---|
| Processing step | Ingredients | % | mg/unit |
| Active layering | Sugar spheres 355-425 µm | 64.6 | 138.62 |
| | Everolimus | 2.3 | 5.00 |
| | Butyl Hydroxy Toluol | 0.05 | 0.10 |
| | Hypromellose 2910 3 cP | 9.3 | 20.00 |
| | Titanium Dioxide | 0.4 | 0.84 |
| | Talc | 1.0 | 2.10 |
| Protection layer coating | Hypromellose 2910 3 cP | 9.0 | 19.23 |
| | Talc | 2.2 | 4.81 |
| | Titan Dioxide | 0.4 | 0.96 |
| Total: | | 100.00 | 191.67 |

**Table 6:**

| Extended release coated multiparticulates Everolimus 5mg, Eudragit RS/RL 1:1, 7.4% polymer weight increase | | | |
|---|---|---|---|
| Processing step | Ingredients | % | mg/unit |
| Top coating | Protection layered pellets 5mg, Table 5 | 89.3 | 191.67 |
| | Eudragit RL 100 | 3.3 | 7.19 |
| | Eudragit RS 100 | 3.3 | 7.19 |
| | Talc | 3.3 | 7.19 |
| | Triethyl citrate | 0.7 | 1.44 |
| Total: | | 100.00 | 214.68 |

### Example 5:

### Extended release pellets for 5mg with use of pore former HPC:

The targeted release profile can be gained by applying a top coating onto protection layered pellets, which contains a certain fraction of pore forming agent. In this example the water soluble polymer hydroxypropyl cellulose was used to form pores in an insoluble ethylcellulose coating. Pellets layered with a matrix containing the active and subsequently layered with a protective layer are produced as described in example 1.

A coating is applied to the protective layered multiparticulates to obtain a product with sustained release properties.

10% lubricant colloidal dioxide and 10% plasticizer triethyl citrate based on amount of polymer are dispersed in ethanol. Then, sustained release polymer ethyl cellulose N-10 (EC) is dissolved with a final concentration of 6 to 7.5% in the solvents. While the dispersion is continuously stirred, HPC (Klucel EF) is added and dissolved at an amount equal to 45% to 50% of the amount of ethyl cellulose.

The protective layered multiparticulates are preheated and fluidized in a fluid bed processor prior to starting spraying the dispersion. The spraying is conducted at a controlled product bed temperature in the range between 35° and 45°C using a bottom spray process until a polymer weight gain of 7.5% to 11 % is received. After finishing the spraying process, the obtained multiparticulates will be dried in the fluid bed at temperatures up to 55°C.

Finally the coated multiparticulates were filled on an automatic capsule filling machine equipped with a dosing chamber filling station into HPMC hard capsules of size 0. The fill weight was adjusted to amount equivalent to 5 mg everolimus.

**Table 7:**

| Extended release coated multiparticulates (EC to pore former HPC ratio: 100:38, 10% weight gain Ethylcellulose) Everolimus 5mg | | | |
|---|---|---|---|
| Processing step | Ingredients | % | mg/unit |
| Top coating | Protection layered pellets 5mg, table 1 | 86.36 | 383.33 |
| | Ethylcellulose N-10 | 8.63 | 38.33 |
| | Hydroxypropylcellulose 300-600 cP | 3.28 | 14.57 |
| | Aerosil 200 | 0.86 | 3.83 |
| | Triethyl citrate | 0.86 | 3.83 |
| Total: | | 100.00 | 443.90 |

**Table 8:**

| Extended release coated multiparticulates (45% pore former HPC, 7.5% weight gain Ethylcellulose) Everolimus 5mg | | | |
|---|---|---|---|
| Processing step | Ingredients | % | mg/unit |
| Top coating | Protection layered pellets 5mg, table 1 | 89.0 | 383.33 |
| | Ethylcellulose N-10 | 6.7 | 28.75 |
| | Hydroxypropylcellulose 300-600 cP | 3.0 | 12.94 |
| | Aerosil 200 | 0.7 | 2.88 |
| | Triethyl citrate | 0.7 | 2.88 |
| Total: | | 100.00 | 430.77 |

**Table 9:**

| Extended release coated multiparticulates (EC to pore former HPC ratio: 100:50, 10.8% weight gain Ethylcellulose) Everolimus 5mg | | | |
|---|---|---|---|
| Processing step | Ingredients | % | mg/unit |
| Top coating | Protection layered pellets 5mg, table 1 | 84.2 | 336.12 |
| | Ethylcellulose N-10 | 9.2 | 36.67 |
| | Hydroxypropylcellulose 300-600 cP | 4.6 | 18.33 |
| | Aerosil 200 | 0.9 | 3.67 |
| | Triethyl citrate | 0.9 | 3.67 |
| | Total: | | 398.46 |

### In-vitro dissolution method:

The multiparticulates were filled into hard capsules of size 0 and then placed into a dissolution vessel filled with 900 mL phosphate buffer pH 6.8 containing sodium dodecyl sulfate 0.2% at 37°C. The dissolution was performed using a paddle method at 75 rpm according to USP monograph 711, and Ph.Eur. monograph 2.9.3. respectively.

### In-vitro dissolution results:

The release profile is shown in figure 1.

| Minutes | % released table 7 | % released table 8 | % released table 9 |
|---|---|---|---|
| 30 | 9.85 | 20.7 | -- |
| 60 | 24.9 | 53.0 | 53.8 |
| 120 | 54.4 | 85.32 | 83.3 |
| 180 | 69.6 | 94.3 | 93.5 |
| 240 | 78.8 | 97.7 | 97.9 |
| 300 | 84.7 | 99.0 | 99.1 |

### Example 6:

### Sustained release pellets for 5mg with use of pore-former HPMC:

Alternatively to example 5, other soluble polymers are also suitable to form pores in insoluble coatings in order to allow for a release of the drug form the pellets. Hypromellose (HPMC) can be used instead of HPC resulting in altered release profile. In this case almost 90% of drug can be released within 2 hours.

Pellets layered with a matrix containing the active and subsequently layered with a protective layer are produced as described in example.

A coating is applied to the protective layered multiparticulates to obtain a product with sustained release properties:
10% lubricant colloidal dioxide and 10% plasticizer triethyl citrate based on amount of polymer are dispersed in ethanol. Then, sustained release polymer ethyl cellulose N-10 is dissolved with a final concentration of 6 to 7.5% in the solvents. While the dispersion is continuously stirred, HPC (Klucel EF) is added and dissolved at an amount equal to 45% to 50% of the amount of ethyl cellulose.

The protective layered multiparticulates are preheated and fluidized in a fluid bed processor prior to starting spraying the dispersion. The spraying is conducted at a controlled product bed temperature in the range between 35° and 45°C using a bottom spray process until a polymer weight gain of 7.5% to 11% is received. After finishing the spraying process, the obtained multiparticulates will be dried in the fluid bed at temperatures up to 55°C.

Finally the coated multiparticulates were filled on an automatic capsule filling machine equipped with a dosing chamber filling station into HPMC hard capsules of size 0. The fill weight was adjusted to amount equivlent to 5 mg everolimus.

**Table 10:**

| Sustained and delayed release coated pellets (EC to pore former HPMC ratio: 100:50, 5% weight gain Ethylcellulose) Everolimus 5mg | | | |
|---|---|---|---|
| Processing step | Ingredients | % | mg/unit |
| Top coating | Protection layered pellets 5mg, Table 4 | 91.7 | 191.67 |
| | Ethylcellulose N-10 | 4.6 | 9.58 |
| | HPMC 2910 3 cP | 2.3 | 4.79 |
| | Aerosil 200 | 0.7 | 1.44 |
| | Triethyl citrate | 0.7 | 1.44 |
| Total: | | 100.00 | 208.92 |

### In-vitro dissolution method:

The multiparticulates were filled into hard capsules of size 0 and then placed into a dissolution vessel filled with 900 mL phosphate buffer pH 6.8 containing sodium dodecyl sulfate 0.2% at 37°C. The dissolution was performed using a paddle method at 75 rpm according to USP monograph 711, and Ph.Eur. monograph 2.9.3., respectively.

### In-vitro dissolution results:

The in-vitro dissolution method as described in example 5 was used.

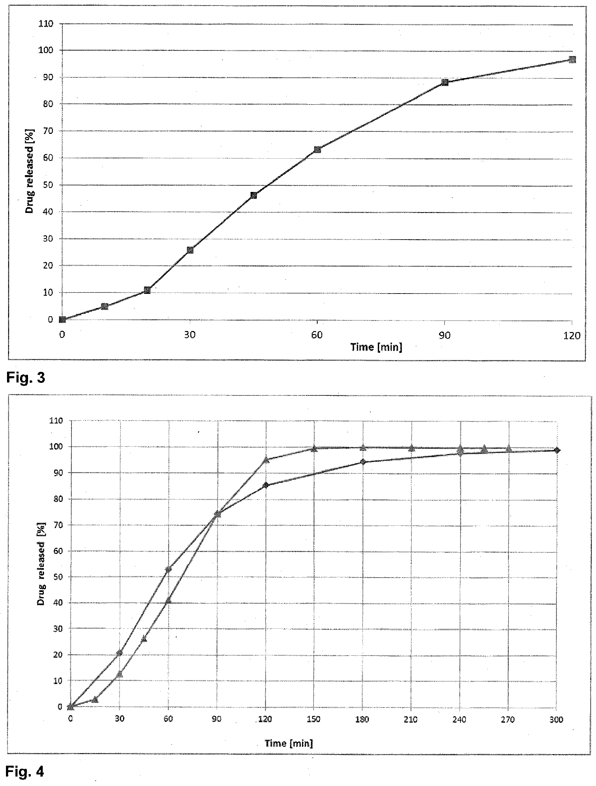

The release profile is shown in figure 3.

| Minutes | % released table 10 |
|---|---|
| 30 | 23.0 |
| 60 | 60.7 |
| 120 | 89.4 |
| 180 | 96.7 |

### Example 7:

### Sustained release minitablets coated with Eudragit RURS:

This example describes a possibility to use minitablets instead of pellets as substrate for extended release coating. A combination of permeable, insoluble polymer Eudragit RS with Eudragit RL was used to achieve retarded release.

A solid dispersion was manufactured with a solvent evaporation process. Solid dispersion consists of everolimus and HPMC 2910 3 cp at ratio of 1:9 parts, and in addition lactose and BHT. The amount of BHT is 2% related to the amount of everolimus.

Everolimus was dissolved in a solvent mixture of ethanol and acetone at a ratio of 1:1, and then subsequently BHT, HPMC and Lactose added to the vessel and suspended. The dispersion was dried in vacuum with drier wall temperature of 50°C.

**Table 11:**

| Everolimus Solid Dispersion 9.09% | | | |
|---|---|---|---|
| Processing step | Ingredients | % | mg/unit |
| Solid dispersion | Everolimus | 9.09 | 5.00 |
| | BHT | 0.18 | 0.10 |
| | Lactose anhydrous | 8.91 | 4.90 |
| | HPMC 29120 3 cP | 81.82 | 45.01 |
| Total: | | 100.00 | 55.01 |

For the manufacture of the minitablets everolimus solid dispersion 9.09%, lactose anhydrous, microcrystalline cellulose and magnesium stearate were mixed with a turbula mixer for 5 minutes. The blend was compressed on a single punch tabletting machine using a minitablet punch tool with 19 punches of 2 mm in diameter. A compression force of approximately 18 kN was applied obtaining mintablets with sufficient tablet hardness of more than 10 N (range: 14-25 N) allowing for coating process.

**Table 12:**

| Minitablets 5mg everolimus | | | |
|---|---|---|---|
| Processing step | Ingredients | % | mg/unit |
| Solid dispersion | Everolimus Solid Dispersion 9.09%, table 11 | 27.5 | 55.01 |
| Tabletting | Lactose anhydrous | 41.5 | 82.99 |
| | Microcrystalline cellulose | 30.0 | 60.00 |
| | Magnesium stearate | 1.0 | 5.00 |
| Total: | | 100.00 | 200.00 |

The minitablets were coated on the lab scale fluid bed coater. A solution of Eudragit RL100 and Eudragit RS100 in a solvent mixture of isopropanol/acetone/water in a ratio of 55.8 : 37.2 : 7.0 was prepared. Plasticizer triethyl citrate and anti tacking agent talc were added. The minitablets were fluidized in the processor with inlet air heated to 27-28°C and coated with a bottom spray process applying a spray pressure of 0.8 Bar.

**Table 13:**

| Sustained release coated minitablets, 5mg everolimus | | | |
|---|---|---|---|
| Processing step | Ingredients | % | mg/unit |
| Top coating | Minitablets everolimus 5mg, table 12 | 89.3 | 191.67 |
| | Eudragit RL 100 | 4.5 | 9.59 |
| | Eudragit RS 100 | 2.2 | 4.79 |
| | Talc | 3.3 | 7.19 |
| | Triethyl citrate | 0.7 | 1.44 |
| Total: | | 100.00 | 214.67 |

### Example 8:

### Sustained release minitablets coated with Ethylcellulose and pore former HPC:

In this example a coating with pore formers was sprayed onto minitablets.

Minitablets were manufacture as descibed for example 7.

A lab scale fluid bed coater was used for a bottom spray coating process. In absolute ethanol the plasticizer triethyl citrate and anti tacking agent colloidal silicon dioxide were dispersed before the coating polymer ethylcellulose N10 and the pore former HPC EF were dissolved. The minitablets were fluidized in the processor with inlet air heated to 43-45°C and coated with spray pressure of 0.8 bar.

**Table 14:**

| Sustained release coated minitablets, EC to pore former HPC ratio 1:1, 7.5% weight gain for Ethylcellulose Everolimus 5mg | | | |
|---|---|---|---|
| Processing step | Ingredients | % | mg/unit |
| Coating | Minitablets everolimus 5mg, table 12 | 82.64 | 200.00 |
| | Ethylcellulose N10 | 6.20 | 15.00 |
| | HPC EF | 6.20 | 15.00 |
| | Triethylcitrate | 1.24 | 3.00 |
| | Aerosil 200 | 3.72 | 9.00 |
| Total: | | 100.00 | 242.00 |

### In-vitro dissolution results:

The in-vitro dissolution method as described in example 5 was used.

The release profile is shown in figure 3.

| Minutes | % released table 14 |
|---|---|
| 30 | 25.8 |
| 60 | 63.2 |
| 90 | 88.4 |
| 120 | 97.0 |

### Example 9:

### 20mg capsule filled with sustained release coated pellets using coating polymer ethylcellulose and pore former HPC:

In this example pellets with higher drug load were used for the filling of hard capsule of size 1 with dose strength of 10 or 20 mg. The product could be improved with respect to its chemical stability by the use of HPMC capsules and the addition of the superdisintegrant crospovidone with high moisture binding capacity.

Pellets layered with a matrix containing the active, and subsequently layered with a protective layer, are produced as described in example 2.

A coating was applied to the protective layered multiparticulates according to process described in example 5. The polymer concentration (EC and HPC) in the spray fluid were set to 10%. The amount of plasticizer HPC and anti tacking agent Aerosil were used at an amount of 10% relative to polymers EC and HPC.

The pellets were filled into HPMC capsule of size 1, and subsequently crospovidone was filled in the same process at a second filling station separately into the capsules.

**Table 15:**

| Extended release coated pellets in capsules (EC to pore former HPC ratio: 100:42, 7.5% weight gain Ethylcellulose) 20mg everolimus | | | |
|---|---|---|---|
| Processing step | Ingredients | % | mg/unit |
| Top coating | Protection layered pellets 20mg, table 2 | 88.7 | 220.00 |
| | Ethylcellulose N-10 | 6.7 | 16.50 |
| | Hydroxypropylcellulose 300-600 cP | 0.9 | 6.93 |
| | Aerosil 200 | 0.9 | 2.34 |
| | Triethyl citrate | 2.8 | 2.34 |
| Capsule filling | Crospovidone | n.a | 50.00 |
| | Capsule shell Qualicaps V (HPMC) size 1 | n.a. | 70.00 |
| Total: | | 100.00 | 368.12 |

### Example 10:

This example demonstrates that it is feasible to use extended release coated pellets as described in the examples above for a tabletting process in order to obtain a tablets as alternative dosage form.

Extended release coated pellets as used in example 9 for filling of hard capsules were alternatively mixed with filler microcrystalline cellulose, glidant colloidal silicon dioxide and lubricrant magnesium stearate in a tumble bin blender to obtain a suitable blend for tabletting. The pellet concentration in the blend was kept at 40% in order to gain a mechanically stable tablet with fully embedded coated pellets. On a single punch machine round, biconvex tablets of 9 mm diameter were compressed with a compression force of 4 kN obataining a tablet hardness of 38 N. The tablets disintegrate fast and the drug release of the tabletted pellets is only marginally impacted by the compaction as it can be seen by dissolution results.

**Table 16:**

| Extended release coated pellets in capsules (EC to pore former HPC ratio: 100:42, 7.5% weight gain Ethylcellulose) 10mg everolimus | | | |
|---|---|---|---|
| Processing step | Ingredients | % | mg/unit |
| Tabletting | Extended Release pellets 10mg Example 9 / table 15 | 40.00% | 107.92 |
| | Microcrystalline Cellulose PH200 | 14.50 | 39.12 |
| | Microcrystalline Cellulose PH102 | 43.50 | 117.36 |
| | Aerosil 200 | 1.00 | 2.70 |
| | Magnesium Stearate | 1.00 | 2.70 |
| Total: | | 100.00 | 269.80 |

### In-vitro dissolution results:

The in-vitro dissolution method as described in example 5 was used.

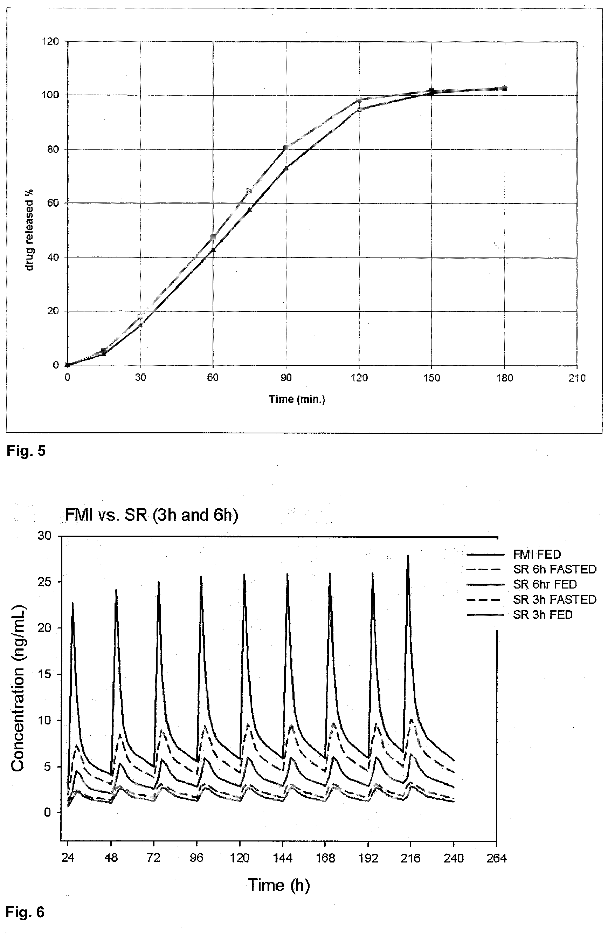

The release profile is shown in figure 6.

| Minutes | Pellets % released table 15 | 10mg tablet % released table 16 |
|---|---|---|
| 30 | 14.8 | 17.9 |
| 60 | 42.9 | 47.4 |
| 120 | 94.9 | 98.4 |
| 180 | 102.9 | 102.5 |

### Example 11:

Extended release profiles can be also achieved by forming diffusion controlled matrix system instead of applying a coating. In this example an extended release matrix is presented where two grades of hypromellose (HPMC) with different viscosities are combined to obtain a swellable, high viscous matrix system with specific release profile.

All amounts of excipients are weighed, sieved and filled into the container of blender, e.g. tumble bin mixer, and are mixed for a suitable time. Magnesium stearat is added not before 5 minutes of the suitable blending time is left to ensure good lubrication during tabletting. The blend was compressed on a single punch tabletting machine using a minitablet punch tool with 19 punches of 2 mm in diameter. A compression force of approximately 12 kN was applied obtaining mintablets with sufficient tablet hardness of more than 15 N.

**Table 17:**

| Extended release matrix minitablets 5 mg everolimus | | | |
|---|---|---|---|
| Processing step | Ingredients | % | mg/unit |
| Blending and Tabletting | Everolimus solid dispersion 9.09% | 22.92 | 55.01 |
| | Methocel K100 LVP CR | 37.50 | 90.00 |
| | Pharmacoat 603 | 12.50 | 30.00 |
| | Lactose anhydrous | 24.58 | 58.99 |
| | Crospovidone XL10 | 1.00 | 2.40 |
| | Colloidal Silicon Dioxide | 0.50 | 1.20 |
| | Magnesium stearate | 1.00 | 2.40 |
| Total: | | 100.00 | 240.00 |

Table 18 (next page): pharmacokinetic parameters from human study comparing 3 different formulations at a single dose of 10mg in fed and fasted state:
- IR:: conventional, immedeate release, fast disintegrating tablet
- SR 3h:: sustained release pellets in a HPMC capsule size 0, 5mg everolimus per capsule, approx. 90% everolimus released in 3h, example 5 / table 8
- SR 6h:: sustained release pellets in a HPMC capsule size 0, 5mg everolimus per capsule approx. 90% everolimus released in 6h, example 5 / table 7

| | | IR | SR 3h FED | SR 3h FAST | SR 6h FED | SR 6h FAST |
|---|---|---|---|---|---|---|
| t_{1/2} | Mean | 36.7 | 38.5 | 37.4 | 37.9 | 46.9 |
| | SD | 6.20 | 7.81 | 11.40 | 13.7 | 21.7 |
| | CV% | 16.9 | 20.3 | 30.5 | 36.1 | 46.2 |
| t ₘₐₓ | Mean | 1.81 | 4.58 | 4.29 | 4.61 | 4.83 |
| | SD | 0.66 | 1.08 | 1.14 | 1.73 | 1.46 |
| | CV% | 36.3 | 23.6 | 26.5 | 37.5 | 30.2 |
| | Range | (1-3) | (3-6) | (2.5-6) | (2.5-8) | (2.5-6) |
| Cₘₐₓ | Mean | 30.16 | 3.61 | 4.29 | 1.91 | 1.76 |
| | SD | 9.58 | 0.946 | 1.14 | 0.488 | 0.452 |
| | CV% | 31.7 | 26.2 | 26.5 | 25.5 | 25.7 |
| C₂₄ₕ | Mean | 2.79 | 1.31 | 1.80 | 0.68 | 0.82 |
| | SD | 0.670 | 0.371 | 0.416 | 0.150 | 0.220 |
| | CV% | 24.0 | 28.3 | 23.2 | 22.1 | 26.8 |
| Cₘₐₓ/C₂₄ₕ | | 10.8 | 2.74 | 2.38 | 2.8 | 2.14 |
| New formulation/FMI % | | | 46.9 | 64.4 | 24.4 | 29.4 |
| AUC_{inf} | Mean | 285.8 | 117.1 | 160.7 | 61.2 | 80.0 |
| | SD | 66.5 | 28.5 | 44.1 | 12.5 | 20.9 |
| | CV% | 23.3 | 24.4 | 27.4 | 20.5 | 26.1 |
| Bioavailability | | | 41.0 | 56.2 | 21.4 | 28.0 |
| Fed vs. Fasted | | | 72.9 | | 76.5 | |

### Figure Legends

Figure 1: in-vitro release profiles of sustained release coated pellets 5mg everolimus in phosphate buffer 6.8, comparison between examples, (Δ) protection layer coated immediate release (example 1/table1), (□) sustained release coated (example 5/table 6), (◊) sustained release coated (example 5/table 8), (○) sustained release coated (example 5/table 9).
Figure 2: in-vitro release profiles of sustained release coated pellets 5mg everolimus in phosphate buffer 6.8, comparison between examples, (Δ) protection layer coated immediate release (example 1/table1), (□) sustained release coated with EC and HPMC as pore former(example 6/ table 10), (◊) sustained release coated with Eudragit RS/RL 3:7(example 3/ table 4).
Figure 3: in-vitro release profiles of sustained release coated minitablets in phosphate buffer 6.8, (□) coated with EC and HPC as pore former (example 8/ table 14).
Figure 4: in-vitro release profiles of sustained release coated pellets coated with EC and HPC as pore former in phosphate buffer 6.8, comparison between examples, (□) 10mg tablet formulation with pellets (example 10 table 16), (Δ) 20mg pellets (example 9/ table 15).
Figure 5: in-vitro release profiles of sustained release coated pellets coated with EC and HPC as pore former in phosphate buffer 6.8, comparison between examples, (◊) 5mg formulation (example 5/ table 8), (Δ) 20mg formulation (example 9/ table 15).
Figure 6: simulation of plasma concentration curve with multiple doses of 10mg everolimus in fed and fasted state comparing 3 different formulations:
   - IR:: conventional, immediate release, fast disintegrating tablet (top line)
   - SR 6h:: sustained release pellets in a HPMC capsule size 0, 5mg everolimus per capsule approx. 90% everolimus relased in 3h, example 5 / table 6 (two bottom lines)
   - SR 3h:: sustained release pellets in a HPMC capsule size 0, 5mg everolimus per capsule, approx. 90% everolimus relased in 3h, example 5 / table 7 (remaining two middle lines)

## Claims

1. An extended release pharmaceutical formulation for oral administration in form of a multiparticulate comprising a) 40-O-(2-hydroxy)ethyl-rapamycin, b) at least one extended release coating which comprises i) a water insoluble coating forming polymer and ii) optionally a pore former, and c) a protection layer, wherein the protection layer separates the layer comprising the 40-O-(2-hydroxy)ethyl-rapamycin from the extended release coating.

2. An extended release pharmaceutical formulation according to claim 1 wherein the pore former is a water soluble cellulose ether, polyethylen glycol, poloxamer 188, povidone, or combinations thereof.

3. An extended release pharmaceutical formulation according to claim 1 or 2 wherein the pore former is hydroxypropylcellulose 300-600cp, HPMC 2910 3 cP, or polyethylen glycol or povidone.

4. An extended release pharmaceutical formulation according to any of the previous claims wherein the coating forming polymer is a water insoluble cellulose ether or cellulose acetate, or polymethacrylates, polyvinylacetate or combinations thereof.

5. An extended release pharmaceutical formulation according to any of the previous claims wherein the coating forming polymer is Eudragit RS or Eudragit RL or a mixture thereof.

6. An extended release pharmaceutical formulation according to any of the previous claims wherein the pore former is water soluble cellulose ether and the coating forming polymer is water insoluble cellulose ether.

7. An extended release pharmaceutical formulation according to any of the previous claims wherein said formulation comprises the 40-O-(2-hydroxy)ethyl-rapamycin in an inner layer with a fast dissolving or disintegrating matrix layer.

8. An extended release pharmaceutical formulation according to claim 7 wherein the fast dissolving or disintegrating matrix layer is placed on a starter core.

9. An extended release pharmaceutical formulation for oral administration in form of a multiparticulate comprising 40-O-(2-hydroxy)ethyl-rapamycin according to claim 1, wherein less than 45% of the active ingredient is released from said pharmaceutical composition after 30 min as determined by a dissolution assay in 900 mL phosphate buffer pH 6.8 containing sodium dodecyl sulfate 0.2% at 37°C and the dissolution is performed using a paddle method at 75 rpm according to Ph.Eur. monograph 2.9.3.

10. An extended release pharmaceutical formulation according to claim 9 the pharmaceutical formulation shows an in-vitro dissolution of component a) of <45% at 0.5h, 20-80% at 1 h, >50% at 2h and >65% at 3h.

11. An extended release pharmaceutical formulation according to claim 10 comprising crospovidone, croscarmellose sodium or sodium starch glycolate as internal desiccant.

12. An extended release pharmaceutical formulation for oral administration in form of a multiparticulate comprising a) 40-O-(2-hydroxy)ethyl-rapamycin according to claim 1, wherein Cₘₐₓ to Cₘᵢₙ ratio of the formulation is <5 at steady state.

13. A solid dosage form comprising an extended release pharmaceutical formulation according to claims 1 to 12 in the form a minitablet, pellets, microparticles, granules or beads.

14. An extended release pharmaceutical formulation according to any one of claims 1 to 12 or a solid dosage form according to claim 13 for use as a medicine.

15. An extended release pharmaceutical formulation for oral administration in a form of a multiparticulate according to claim 1 for use in a method of reducing everolimus Cₘₐₓ to Cₘᵢₙ ratio in a patient.

## Patentansprüche

1. Pharmazeutische Formulierung mit verlängerter Freisetzung zur oralen Verabreichung in Form eines Multipartikulats, umfassend a) 40-0-(2-hydroxy)ethyl-rapamycin, b) mindestens eine Beschichtung mit verlängerter Freisetzung, die i) ein Polymer, das eine wasserunlösliche Beschichtung bildet, und ii) gegebenenfalls einen Porenbildner umfasst, und c) eine Schutzschicht, wobei die Schutzschicht die 40-O-(2-hydroxy)ethyl-rapamycin-umfassende Schicht von der Beschichtung mit verlängerter Freisetzung trennt.

2. Pharmazeutische Formulierung mit verlängerter Freisetzung nach Anspruch 1, wobei der Porenbildner ein wasserlöslicher Celluloseether, Polyethylenglycol, Poloxamer 188, Povidon oder Kombinationen davon ist.

3. Pharmazeutische Formulierung mit verlängerter Freisetzung nach Anspruch 1 oder 2, wobei der Porenbildner Hydroxypropylcellulose 300-600cp, HPMC 2910 3 cP oder Polyethylenglycol oder Povidon ist.

4. Pharmazeutische Formulierung mit verlängerter Freisetzung nach einem der vorhergehenden Ansprüche, wobei das beschichtungsbildende Polymer ein wasserunlöslicher Celluloseether oder Celluloseacetat, ein Polymethacrylat, Polyvinylacetat oder Kombinationen davon ist.

5. Pharmazeutische Formulierung mit verlängerter Freisetzung nach einem der vorhergehenden Ansprüche, wobei das beschichtungsbildende Polymer Eudragit RS oder Eudragit RL oder ein Gemisch davon ist.

6. Pharmazeutische Formulierung mit verlängerter Freisetzung nach einem der vorhergehenden Ansprüche, wobei der Porenbildner ein wasserlöslicher Celluloseether und das beschichtungsbildende Polymer ein wasserunlöslicher Celluloseether ist.

7. Pharmazeutische Formulierung mit verlängerter Freisetzung nach einem der vorhergehenden Ansprüche, wobei die Formulierung 40-O-(2-hydroxy)ethyl-rapamycin in einer inneren Schicht mit einer schnellauflösenden oder zerfallbaren Matrixschicht umfasst.

8. Pharmazeutische Formulierung mit verlängerter Freisetzung nach Anspruch 7, wobei die schnellauflösende oder zerfallbare Matrixschicht auf einem Starterkern vorliegt.

9. Pharmazeutische Formulierung mit verlängerter Freisetzung zur oralen Verabreichung in Form eines Multipartikulats, umfassend 40-0-(2-hydroxy)ethyl-rapamycin nach Anspruch 1, wobei weniger als 45% des Wirkstoffs nach 30 Minuten von der pharmazeutischen Zusammensetzung freigesetzt werden, bestimmt mittels eines Lösungsassays in 900 mL Phosphatpuffer (pH-Wert = 6.8) enthaltend Natriumdodecylsulfat (0,2%) bei 37°C, wobei die Lösung unter Verwendung einer Rührblattmethode bei 75 U/min gemäß Ph.Eur. Monograph 2.9.3 durchgeführt wird.

10. Pharmazeutische Formulierung mit verlängerter Freisetzung nach Anspruch 9, wobei die pharmazeutische Formulierung eine *in-vitro*-Auflösung der Komponente a) von <45% bei 0,5 Stunden, 20-80% bei einer Stunde, >50% bei 2 Stunden und >65% bei 3 Stunden aufweist.

11. Pharmazeutische Formulierung mit verlängerter Freisetzung nach Anspruch 10, umfassend Crospovidon, Croscarmellose-Natrium oder Natriumstärkeglycolat als internes Trockenmittel.

12. Pharmazeutische Formulierung mit verlängerter Freisetzung zur oralen Verabreichung in Form eines Multipartikulats umfassend a) 40-0-(2-hydroxy)ethyl-rapamycin nach Anspruch 1, wobei das Verhältnis von Cₘₐₓ zu Cₘᵢₙ der Formulierung im Gleichgewicht <5 ist.

13. Feste Dosierungsform, umfassend eine pharmazeutische Formulierung mit verlängerter Freisetzung gemäß der Ansprüche 1 bis 12 in Form einer/s Minitablette, Pellets, Mikropartikels, Granulats oder Kügelchens.

14. Pharmazeutische Formulierung mit verlängerter Freisetzung nach einem der Ansprüche 1 bis 12 oder eine feste Dosierungsform nach Anspruch 13 zur Verwendung als Medikament.

15. Pharmazeutische Formulierung mit verlängerter Freisetzung zur oralen Verabreichung in Form eines Multipartikulats nach Anspruch 1 zur Verwendung in einem Verfahren zum Verringern des Everolismus Cₘₐₓ zu Cₘᵢₙ Verhältnisses in einem Patienten.

## Revendications

1. Formulation pharmaceutique à libération prolongée pour une administration orale sous forme multiparticulaire comprenant a) de la 40-O-(2-hydroxy)éthyl-rapamycine, b) au moins un enrobage à libération prolongée qui comprend i) un polymère formant un enrobage insoluble dans l'eau et ii) éventuellement un agent porogène, et c) une couche de protection, dans laquelle la couche de protection sépare la couche comprenant la 40-O-(2-hydroxy)éthyl-rapamycine de l'enrobage à libération prolongée.

2. Formulation pharmaceutique à libération prolongée selon la revendication 1 dans laquelle l'agent porogène est un éther cellulosique soluble dans l'eau, un polyéthylène glycol, le poloxamère 188, la polyvidone, ou leurs combinaisons.

3. Formulation pharmaceutique à libération prolongée selon la revendication 1 ou 2 dans laquelle l'agent porogène est l'hydroxypropylcellulose de 300 à 600 cP, la HPMC 2910 de 3 cP, ou un polyéthylène glycol ou la polyvidone.

4. Formulation pharmaceutique à libération prolongée selon l'une quelconque des revendications précédentes, dans laquelle le polymère formant un enrobage est un éther cellulosique insoluble dans l'eau ou l'acétate de cellulose, ou des polyméthacrylates, ou un polyacétate de vinyle ou leurs combinaisons.

5. Formulation pharmaceutique à libération prolongée selon l'une quelconque des revendications précédentes, dans laquelle le polymère formant un enrobage est de l'Eudragit RS ou de l'Eudragit RL ou un mélange de ceux-ci.

6. Formulation pharmaceutique à libération prolongée selon l'une quelconque des revendications précédentes, dans laquelle l'agent porogène est un éther cellulosique soluble dans l'eau et le polymère formant un enrobage est un éther cellulosique insoluble dans l'eau.

7. Formulation pharmaceutique à libération prolongée selon l'une quelconque des revendications précédentes, où ladite formulation comprend la 40-O-(2-hydroxy)éthyl-rapamycine dans une couche interne avec une couche de matrice à dissolution ou désintégration rapide.

8. Formulation pharmaceutique à libération prolongée selon la revendication 7 dans laquelle la couche de matrice à dissolution ou désintégration rapide est placée sur un noyau de départ.

9. Formulation pharmaceutique à libération prolongée pour une administration orale sous forme multiparticulaire comprenant de la 40-O-(2-hydroxy)éthyl-rapamycine selon la revendication 1, dans laquelle moins de 45 % du principe actif est libéré à partir de ladite composition pharmaceutique après 30 min comme il est déterminé par un test de dissolution dans 900 ml de tampon phosphate pH 6,8 contenant du dodécylsulfate de sodium à 0,2 % à 37 °C et la dissolution est effectuée en utilisant un procédé à palettes à 75 tr/min selon la monographie 2.9.3. de la Pharmacopée Européenne.

10. Formulation pharmaceutique à libération prolongée selon la revendication 9, dans laquelle la formulation pharmaceutique présente une dissolution *in vitro* du composant a) < 45 % à 0,5 h, de 20 à 80 % à 1 h, > 50 % à 2 h et > 65 % à 3 h.

11. Formulation pharmaceutique à libération prolongée selon la revendication 10 comprenant de la polyvinylpolypyrrolidone, de la croscarmellose sodique ou du glycolate d'amidon sodique en tant que dessiccateur interne.

12. Formulation pharmaceutique à libération prolongée pour une administration orale sous forme microparticulaire comprenant a) de la 40-O-(2-hydroxy)éthyl-rapamycine selon la revendication 1, dans laquelle le rapport Cₘₐₓ sur Cₘᵢₙ de la formulation est < 5 à l'état d'équilibre.

13. Forme galénique solide comprenant une formulation pharmaceutique à libération prolongée selon les revendications 1 à 12 sous la forme d'un mini-comprimé, de granules, de microparticules, de granulés ou de billes.

14. Formulation pharmaceutique à libération prolongée selon l'une quelconque des revendications 1 à 12 ou forme galénique solide selon la revendication 13 pour une utilisation en tant que médicament.

15. Formulation pharmaceutique à libération prolongée pour une administration orale sous forme multiparticulaire selon la revendication 1 pour une utilisation dans un procédé de réduction du rapport Cₘₐₓ sur Cₘᵢₙ de l'éverolimus chez un patient.
